# EUROPÄISCHE PATENTSCHRIFT

(11) **EP 2 353 493 B1**
(45) Veröffentlichungstag und Bekanntmachung des Hinweises auf die Patenterteilung: **30.03.2016**
(21) Anmeldenummer: 11152642.2
(22) Anmeldetag: 31.01.2011
(51) Int. Cl.: A61B 1/00, A61B 1/045, F41G 1/00, G02B 23/00

(54) **Medizinisches Instrument mit einem magnetischen Stelltrieb**
Medical instrument with a magnetic actuator
Instrument médical doté d'un moteur électrique magnétique

(30) Priorität: 01.02.2010 DE 102010007394
(43) Veröffentlichungstag der Anmeldung: 10.08.2011
(73) Patentinhaber: Karl Storz GmbH & Co. KG, 78532 Tuttlingen (DE)
(72) Erfinder: Dahmen, Jan, 78606, Seitingen-Oberflacht (DE)
(74) Vertreter: Witte, Weller & Partner Patentanwälte mbB

(56) Entgegenhaltungen:
- EP-A1- 1 767 137
- DE-A1- 4 211 203
- DE-A1- 19 927 816
- GB-A- 789 001
- US-A1- 2007 010 707

## Beschreibung

Die Erfindung betrifft ein medizinisches Instrument, mit einem zumindest einen Magneten aufweisenden äußeren Stelltrieb, der mit einer im Inneren des medizinischen Instrumentes angeordneten, bewegbaren, zumindest einen Magneten aufweisenden inneren Einheit derart magnetisch gekoppelt ist, dass ein Bewegen des äußeren Stelltriebes eine Bewegung der inneren bewegbaren Einheit verursacht, wobei der zumindest eine Magnet des äußeren Stelltriebes sowohl axial verschiebbar als auch drehbar ausgebildet ist, und dass ferner die innere bewegbare Einheit sowohl axial verschiebbar als auch drehbar ausgebildet ist, und dass die magnetische Kopplung zwischen dem äußeren Stelltrieb und der inneren Einheit derart ausgebildet ist, dass eine Rotation des äußeren Stelltriebes eine Rotation der inneren Einheit bewirkt, und dass eine axiale Verschiebung des zumindest einen Magneten desselben Stelltriebes eine axiale Verschiebung der inneren Einheit verursacht.

Ein derartiges medizinisches Instrument ist aus der US 2007/0010707 A1 bekannt.

Derartige Magnettriebe sind außerhalb des medizinischen Bereiches bspw. aus der DE 42 11 203 A1 in Zusammenhang mit der Innenfokussierung von Feldstechern, Zielfernrohren und dergleichen oder aus der GB 789 001 A in Zusammenhang mit Teleskopen bekannt.

Derartige Magnettriebe dienen dazu, mittels eines von der Außenseite betätigbaren äußeren Stellantriebes, der einen Magneten aufweist, über Magnetkopplung eine innere Einheit, die ebenfalls zumindest einen Magneten aufweist, zu bewegen.

Dies eröffnet die Möglichkeit, die innere Einheit hermetisch dicht zur Außenseite abzuschließen, da keinerlei mechanische Verbindungen zwischen dem äußeren Stelltrieb und der inneren bewegbaren Einheit erforderlich sind. Die Kraftübertragung von dem äußeren Stelltrieb auf die innere bewegbare Einheit erfolgt durch Magnetkraft bzw. die Kopplung der äußeren und inneren Magnete.

Beispielsweise ist ein derartiges Instrument aus der DE 197 13 276 C2 bekannt.

Dort besteht der äußere Stelltrieb aus einem äußeren Drehring, der einen inneren Drehring, der die innere Einheit trägt, dreht. Beide Drehringe sind axial zueinander unverschiebbar. Mit diesem Magnettrieb können ausschließlich Drehbewegungen des äußeren Stelltriebes in eine Drehbewegung der inneren Einheit umgesetzt werden. Dies dient beispielsweise dazu, um optische Elemente wie Filter, Blenden oder dgl. in einen optischen Pfad ein- und auszuschwenken.

Ein anderes medizinisches Instrument mit einem derartigen Magnettrieb ist aus der DE 195 21 654 A1 bekannt. Auch hier weist der äußere Stelltrieb einen äußeren Drehring auf, der axial unverschiebbar angeordnet ist.

Die innere Einheit ist auf einer schraubenlinienförmigen Bahn geführt, so dass ein Drehen des äußeren Drehrings durch Drehen der inneren Einheit längs der schraubenlinienförmigen Bahn in eine axiale Verschiebung umgesetzt wird. Derartige axiale Verschiebungen dienen z.B. dazu, ein optisches System zu fokussieren. Hier wird also die Drehbewegung des äußeren Stelltriebes in eine axiale Bewegung der inneren Einheit umgesetzt.

Aus der US-A-5 539 992 ist ein medizinisches Instrument bekannt, bei dem der äußere Stelltrieb ein äußeres Ringelement aufweist, in dem zwei schraubenlinienförmige Schlitze ausgespart sind, in denen Magnete laufen. Diese äußeren Magnete stehen mit einer axialen Nut einer Hülse in Verbindung. Ein Drehen des äußeren Ringelementes des äußeren Stellantriebes verursacht somit eine axiale Verschiebung der äußeren Magnete. Diese bewegen über eine magnetische Kopplung die Magnete einer inneren Einheit in axialer Richtung.

Aus der DE 88 10 044 U1 ist eine optische Einstellvorrichtung bekannt, bei der ein äußerer Ring einen Magneten trägt, der mit einem lang erstreckten Magneten im Innern zusammenwirkt. Der äußere Ring ist so ausgestaltet, dass ein Drehen des Ringes dessen axiale Verschiebung bewirkt, so dass der von diesem Ring getragene Magnet den in der inneren Einheit aufgenommenen Magneten axial bewegt.

Nachteilig an den zuvor genannten Ausgestaltungen von Magnettrieben ist, dass diese so ausgestaltet sind, dass sie jeweils nur eine bestimmte Bewegung der inneren Einheit verursachen können, also entweder ein Drehen oder ein axiales Verschieben der inneren Einheit.

Es ist Aufgabe der vorliegenden Erfindung, ein medizinisches Instrument mit einem Magnettrieb dahingehend weiterzuentwickeln, dass eine besonders feine Justierung der inneren Einheit bewerkstelligbar ist.

Erfindungsgemäß wird die Aufgabe dadurch gelöst, dass der axiale Verschiebeweg des äußeren Stelltriebes über eine Übersetzung in einen relativ dazu gesehen kürzeren Verschiebeweg der inneren Einheit umsetzbar ist, dass zwischen einer Schräge der inneren Einheit und einer Schräge einer umgebenden Hülse zumindest eine Kugel angeordnet ist, die radial zwischen den Schrägen bewegbar ist, und dass die Steigung der Schräge ein Übersetzungsverhältnis zwischen axialem Verschiebeweg des äußeren Stelltriebes und einem axialen Verschiebeweg der inneren Einheit bestimmt.

Bei der Erfindung ist der axiale Verschiebeweg des äußeren Stelltriebes über eine Übersetzung in den Verschiebeweg der inneren Einheit umsetzbar.

Durch die Zwischenschaltung einer Übersetzung, oder je nach Blickrichtung einer Untersetzung, ist es möglich, beispielsweise einen relativ langen Verschiebeweg des äußeren Stelltriebes in einen relativ kurzen Verschiebeweg des inneren Stelltriebes umzusetzen. In anderen Worten ausgedrückt, können relativ lange Teilschritte des äußeren Stelltriebes in relativ kurze Verschiebewege der inneren Einheit umgesetzt werden, so dass dann eine besonders feine Justierung der inneren Einheit zwischen den maximalen Endpositionen erfolgen kann. Bei Fokussierung einer optischen Vorrichtung können somit auch Scharfstellungen in den Zwischenbereichen zwischen Fernbereich und Nahbereich erzielt werden.

Bei der Erfindung ist zwischen einer Schräge der inneren Einheit und einer Schräge einer umgebenden Hülse zumindest eine Kugel angeordnet, die radial zwischen den Schrägen bewegbar ist.

Zum einen kann durch Auswahl eines bestimmten Anpressdruckes zwischen den Schrägen bzw. einer entsprechenden Anwinklung der Schrägen der Laufweg der Kugel bzw. auch der mehreren umfänglich verteilten Kugeln gehemmt werden, so dass das System an einem bestimmten axialen Verschiebeweg selbsthemmend gehalten wird.

Bei der Erfindung wird durch Variierung der Steigung der Schräge ein Übersetzungsverhältnis zwischen axialem Verschiebeweg des äußeren Stelltriebes und einem axialen Verschiebeweg der inneren Einheit bestimmt.

Dies eröffnet die Möglichkeit, durch die Änderung des Winkels der Schrägen ein bestimmtes Übersetzungsverhältnis einzustellen.

Steht beispielsweise die Schräge der inneren Einheit gegenüber der Längsachse unter einem Winkel von 45° und wird die Kugel gegen eine sich exakt rechtwinklig zur Mittellängsachse erstreckenden Schulter gedrückt, so entspricht der radiale Verschiebeweg der Kugel beim seitlichen Ausdrücken exakt dem axialen Verschiebeweg der inneren Einheit.

In anderen Worten ausgedrückt wird die Bewegung des äußeren Stelltriebes zur axialen Verstellung der inneren Einheit im Verhältnis 1:1 umgesetzt. Wird die Schräge unter einen anderen Winkel gestellt, kann dann ein entsprechend anderes Übersetzungs- oder Untersetzungsverhältnis gewählt werden, je nachdem, wie das gewünscht ist. In Zusammenhang mit der Feder kann der Anpressdruck entsprechend gewählt werden, so dass dann auch die Halterung oder Selbsthemmung des Systems dadurch beeinflusst werden kann.

Durch diese Ausgestaltung ist es möglich, mit ein und demselben äußeren Stelltrieb die innere Einheit sowohl zu drehen als auch axial zu verschieben, und zwar unabhängig voneinander.

Unabhängig bedeutet, dass durch Betätigen des äußeren Stelltriebes nur eine axiale Verschiebung der inneren Einheit bewirkt werden kann, um beispielsweise einen Fokussiervorgang durchzuführen. Der äußere Stelltrieb ist aber auch so zu betätigen, dass nur ein Drehen der inneren Einheit durchgeführt wird, ohne dass dabei gleichzeitig ein axiales Verschieben der inneren Einheit stattfindet.

Ein Drehen der inneren Einheit ist beispielsweise bei Videoendoskopen zur Bildaufrichtung erwünscht, insbesondere wenn das Objektiv eine von der Mittellängsachse des Schaftes abgewinkelte Blickrichtung erlaubt.

Wird ein solches Videoendoskop als gesamtes gedreht, dreht sich auch das erfasste Bild und es besteht die Gefahr, dass man die Orientierung in dem Hohlorgan verliert. Beispielsweise bei der Inspektion einer Blase als Hohlorgan kann dies vorkommen.

Es wird daher von den Ärzten gewünscht, dass eine Möglichkeit der Aufrichtung eines durch ein solches Videoendoskop aufgenommenen Bildes in einer bestimmten Nord-Süd-Ausrichtung möglich ist. Dies kann durch den äußeren Stelltrieb erfolgen, indem dieser so betätigt wird, dass ausschließlich die innere Einheit gedreht wird. Dadurch ist dann auch sichergestellt, dass die zuvor eingestellte axiale Verschiebung, also eine Fokussierung, um ein scharfes Bild zu erhalten, weiter bestehen bleibt. Es ist auch möglich, nachdem beispielsweise die innere Einheit zur Bildaufrichtung gedreht wurde, nachzujustieren oder nachzufokussieren, wenn das wieder aufgerichtete Bild beispielsweise anstatt eines Nahbereichbildes ein Bild aus größerer Entfernung darstellen soll. Dann kann durch Betätigen des äußeren Stellantriebes derart, dass nur eine axiale Verschiebung der inneren Einheit erfolgt, dieser Fokussiervorgang durchgeführt werden.

Daraus ergibt sich eine sehr hohe Flexibilität bei der Handhabung von medizinischen Instrumenten, die insbesondere bei optischen Systemen ein Justieren durch axiale Verschiebung der inneren Einheit erlaubt.

Die axiale Verschiebung der inneren Einheit ist nicht auf Fokussiervorgänge beschränkt, es können auch andere Vorgänge durchgeführt werden, beispielsweise ein Ausfahren einer Messsonde oder eines Mess- oder Abtaststabes. Insgesamt gesehen wird daher die Flexibilität der Handhabbarkeit eines solchen medizinischen Instruments und dessen Einsatzbereich erheblich erweitert.

Dadurch wird die Aufgabe vollkommen gelöst.

In einer Ausgestaltung der Erfindung weist der äußere Stelltrieb ein einziges Stellelement auf, das sowohl drehbar als auch axial verschiebbar ist.

Diese Maßnahme hat den Vorteil, dass durch eine konstruktiv sehr einfache Ausgestaltung sowohl die Drehung der inneren Einheit als auch deren axiale Verschiebung bewirkt werden kann.

Dies erfolgt dadurch, dass man den äußeren Stelltrieb zum Drehen der inneren Einheit verdreht und zum Bewegen der inneren Einheit in axialer Richtung entsprechend axial hin und her verschiebt.

Ein Drehen des äußeren Stelltriebes kann bis nahezu 360° erforderlich sein, zumindest in jeder Richtung etwa um 180°, um ein beispielsweise auf dem Kopf stehendes Bild wieder in die gewünschte Nord-Süd-Ausrichtung aufzurichten.

Der axiale Verschiebeweg, beispielsweise wenn es sich nur um eine Fokussierung handelt, kann relativ kurz sein, er kann auch lang sein, wenn beispielsweise durch das axiale Verschieben Sonden ausgefahren oder sonstige Bewegungsvorgänge erwünscht sind. Dies zeigt die hohe Flexibilität und die Erweiterung des Einsatzbereiches eines solchen Magnettriebes.

Wenn mit einem einzigen Stelltrieb beide Bewegungen durchgeführt werden können, kann man prinzipiell auch diese Bewegungen gleichzeitig durchführen. Das kann manchmal erwünscht sein. Wenn es aber unerwünscht ist, muss man entsprechende Vorkehrungen treffen, die beispielsweise ein versehentliches axiales Verschieben bei einem Drehen durch die Handhabungsperson ausschließt. Dies kann beispielsweise dadurch erfolgen, dass der gesamte Stelltrieb relativ schwergängig gemacht wird, so dass die Kräfte ganz gezielt in die eine oder in die andere Richtung gerichtet auf den Stelltrieb einwirken müssen.

In einer weiteren Ausgestaltung der Erfindung ist vorgesehen, dass der äußere Stelltrieb zwei Stellelemente aufweist, ein erstes Stellelement zum Bewirken der Rotation der inneren Einheit und ein zweites Stellelement zum Bewirken der axialen Verschiebung der inneren Einheit.

Diese Maßnahme hat den Vorteil, dass die zuvor erwähnten Probleme dahingehend ausgeschaltet werden können, dass zwar nach wie vor mit ein und demselben äußeren Stelltrieb die Magnete bewegt werden, dass aber zwei unterschiedliche Stellelemente für Drehen und axiales Verschieben vorhanden sind. Das kann man prinzipiell dann so ausgestalten, dass das eine Stellelement nur axial hin und her verschoben werden kann, um dadurch die axiale Verschiebung zu bewirken, das andere Stellelement aber nur gedreht werden kann. Dies kann der Handhabungsperson beispielsweise einfach dadurch angezeigt werden, dass die beiden Stellelemente unterschiedlich ausgebildet sind, oder mit entsprechenden Markierungen versehen, die die Verstellrichtung, die dieses Stellelement bewirkt, eindeutig anzeigt, beispielsweise in Form von Richtungspfeilen.

In einer weiteren Ausgestaltung der Erfindung ist das zweite Stellelement zum Bewirken der axialen Verschiebung der inneren Einheit drehbar ausgebildet und steht mit einem hülsenartigen Körper in Verbindung, der die Drehbewegung des zweiten Stellelements in eine axiale Verschiebung des zumindest einen Magneten des Stelltriebes verursacht.

Diese Maßnahme hat den Vorteil, dass beide Verstellbewegungen, also sowohl Drehbewegung der inneren Einheit als auch deren axiale Verschiebung durch Drehbewegungen der äußeren Stellelemente verursacht wird.

Manche Handhabungspersonen und Ärzte sind es gewohnt, beispielsweise Fokussiervorgänge mit einer axialen Verschiebung einer inneren Einheit durch Drehen eines äußeren Fokussierringes durchzuführen.

Um diesem Habitus Rechnung zu tragen, sind nun die Stellelemente so ausgebildet, dass sie beide drehbar sind. Die Drehung des einen Stellelementes wird unmittelbar zum Drehen der inneren Einheit herangezogen. Die Drehbewegung des anderen Stellelementes wird durch Zwischenschaltung des hülsenartigen Körpers in eine lineare Bewegung des zumindest einen Magneten des äußeren Stelltriebes umgesetzt, um dann durch diesen die innere Einheit axial zu verschieben.

In einer weiteren Ausgestaltung der Erfindung ist das erste Stellelement derart am zweiten Stellelement angebracht, dass ein Drehen des ersten Stellrades ein Drehen der inneren Einheit aus jeder beliebigen axialen Verschiebestellung des äußeren Stelltriebes verursacht.

Diese Maßnahme hat den Vorteil, dass beispielsweise die beiden Stellelemente eng nebeneinander liegend angeordnet werden können, ohne dass sie sich bei der Drehbewegung voneinander weg bewegen. Die Drehbewegung des zweiten Stellelementes, das für die axiale Verschiebung dient, wird ja durch eine Hülse in einen linaren Verschiebeweg des zumindest einen Magneten des äußeren Stelltriebes umgesetzt. Das erste Stellelement kann dann in jeder beliebigen Stellung benutzt werden, um den äußeren Stelltrieb zu verdrehen und dadurch die innere Einheit zu drehen, und zwar unabhängig davon, in welcher axialen Verschiebestellung sie sich gerade befindet.

In einer weiteren Ausgestaltung der Erfindung ist die innere Einheit zwischen zwei Anschlägen axial hin und her bewegbar, wobei der axiale Verschiebeweg des äußeren Stelltriebes um ein Mehrfaches länger ist als der Verschiebeweg zwischen den beiden Anschlägen.

Diese Maßnahme hat den Vorteil, dass die innere Einheit gefedert an die Endanschläge gedrückt wird. Das Magnetkopplungsprinzip ist ja so, dass die Magnete der inneren Einheit der Bewegung der Magnete des äußeren Stelltriebes folgen.

Ist nun der axiale Verschiebeweg der äußeren Magnete wesentlich länger, so würde ja ohne die Anschläge die innere Einheit auch über den längeren axialen Verschiebeweg bewegt werden, um den die äußeren Magnete bewegt werden.

Fahren aber die inneren Magnete schon vor Ende des Verschiebeweges jeweils in beiden Richtungen gegen einen Anschlag, so verursacht ein weiteres Verschieben der äußeren Magnete in axialer Richtung, dass die innere Einheit gegen diese Anschläge gedrückt wird, somit besonders fest in diesen Endstellungen gehalten wird. Dies ist durch die Magnetkopplung gefedert, da ja die äußeren Magnete dauernd dazu tendieren, die inneren Magnete über die Anschläge hinaus zu bewegen.

Das bedeutet beispielsweise, dass bei mechanischen Schocks, sei es durch unsachgemäße Handhabung oder beispielsweise bei Herabfallen eines Gerätes, die innere Einheit sich etwas bewegen kann, durch den "Magnetdruck", aber an den Anschlägen gehalten oder wieder dorthin zurückbewegt wird.

Diese Ausgestaltung ist insbesondere vorteilhaft, wenn definierte Fokussierwege vorhanden sind und die eine oder andere Fokussierendstellung, meist "Nahbereich" oder "Fernbereich", fest eingehalten werden soll.

Durch diese Maßnahme wird sichergestellt, dass die innere Einheit jeweils in der einen oder anderen Stellung fest und federnd gehalten wird.

In einer weiteren Ausgestaltung der Erfindung weist die innere bewegbare Einheit eine Hülse auf, die den zumindest einen Magneten der inneren Einheit trägt.

Diese Maßnahme hat den Vorteil, dass durch den hülsenartigen Aufbau die Möglichkeit besteht, einen oder mehrere Magnete an der inneren Einheit an einer jeweils günstigen Stelle anzubringen oder zu verankern, indem beispielsweise an der Außenseite der Hülse entsprechende Vertiefungen vorgesehen werden.

Zugleich bleibt die Möglichkeit eröffnet, durch den Innenraum der Hülse hindurch Bauelemente des medizinischen Instrumentes hindurchzuführen. Bei einem Endoskop können das Bauelemente des optischen Pfades sein.

Bei klassischen Endoskopen können diese optischen Elemente Linsen oder dgl. sein, bei Videoendoskopen entsprechende Leitungen, Kabel oder dgl.

In einer weiteren Ausgestaltung der Erfindung weist der äußere Stelltrieb ein Stellrad auf, das zumindest einen Magneten trägt, und dieses Stellrad umgibt die Hülse der inneren Einheit.

Diese an sich bekannte Maßnahme erlaubt eine sehr intensive magnetische Kopplung, indem beispielsweise die Magnete an der Innenseite des Stellrades sehr nah zu den Magneten an der Außenseite der Hülse montiert werden können. Andererseits kann die innere Einheit als eine zur Außenseite hermetisch abgeschlossene kompakte Einheit ausgebildet werden und in den Innenraum des Stellrades des äußeren Stelltriebes eingebracht bzw. montiert werden. Dies erlaubt es, Autoklaviervorgänge mit dem Instrument durchzuführen.

In einer weiteren Ausgestaltung der Erfindung sind an der inneren Einheit umfänglich und axial verteilt mehrere Magnete angeordnet, und auch am äußeren Stelltrieb sind ebenfalls umfänglich und axial verteilt mehrere Magnete angeordnet, wobei sich gleiche Pole der inneren und äußeren Magnete gegenüberstehen.

Diese Maßnahme hat den Vorteil, dass durch die Abstoßung eine Zentrierung der inneren Einheit in dem äußeren Stelltrieb erfolgt. Dies fördert besonders die Leichtgängigkeit des Magnettriebes und unterstützt einen besonders ruhigen zentrischen bzw. kozentrischen Lauf des Magnettriebes.

In einer weiteren Ausgestaltung der Erfindung erfolgt die axiale Verschiebung der inneren Einheit gegen einen Haltemechanismus, der die innere Einheit in jeder beliebigen axialen Verschiebestellung hält.

Diese Maßnahme hat den Vorteil, dass über den Haltemechanismus die innere Einheit in jeder beliebigen axialen Verschiebestellung gehalten werden kann.

Wie bereits mehrfach erwähnt, wird ja die axiale Verschiebung der inneren Einheit meist zu Fokussiervorgängen herangezogen. Wenn gewünscht ist, nicht nur zwischen zwei extremen Endstellungen, also wie beispielsweise zuvor erwähnt "Fernbereich" oder "Nahbereich", hin und her zu bewegen, sondern auch in Zwischenstellungen zu halten, kann dies durch den zusätzlichen Haltemechanismus erfolgen. Dieser Haltemechanismus kann nun unterschiedlich ausgestaltet sein, beispielsweise einfach durch eine Schwergängigkeit der axialen Verschiebung, so dass dadurch die innere Einheit quasi selbsthemmend ist. Der äußere Stelltrieb muss natürlich so ausgebildet sein, dass er diese Selbsthemmung überwinden kann und die axiale Verschiebung der inneren Einheit bewirkt. Wird der äußere Stelltrieb freigegeben, wird durch den Haltemechanismus die innere Einheit in der jeweiligen axialen Verschiebeposition zwischen den Endpositionen gehalten.

In einer weiteren vorteilhaften Ausgestaltung ist vorgesehen, dass, indem die Schräge der inneren Einheit mit Federkraft beaufschlagbar ist, zumindest eine Kugel gegen eine Schulter drückbar ist.

Diese Maßnahme hat den Vorteil, dass, indem die Schräge mit der Federkraft beaufschlagt ist, die Kugeln gegen die Schulter gedrückt werden, die eine axiale Bewegung derselben in eine Richtung sperrt, gleichzeitig die Kugel aber zwischen den Schrägen der inneren Einheit und der anliegenden Außenhülse bei deren relativer axialen Verschiebung radial bewegbar ist. Dadurch kann eine Kraftverstärkung erzielt werden.

Zusammenfassend ist zu sagen, dass durch diese Ausgestaltungen die Variabilität und der Einsatzbereich des medizinischen Instrumentes weiter erhöht wird, insbesondere wenn eine hochgenaue Verschiebung der inneren Einheit in axialer Richtung über einen relativ kurzen Weg gewünscht wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Ansicht eines ersten Ausführungsbeispiels eines medizinischen Instrumentes mit einem Magnettrieb mit nur einem Stellelement,
- Fig. 2: einen abschnittsweisen Längsschnitt des medizinischen Instrumentes von Fig. 1 in einer axialen Endposition des äußeren Stelltriebes,
- Fig. 3: eine der Fig. 2 entsprechende Darstellung in der entgegengesetzten Endposition des äußeren Stelltriebes,
- Fig. 4: eine perspektivische Darstellung eines zweiten Ausführungsbeispieles eines medizinischen Instrumentes, das mit einem erfindungsgemäßen Magnettrieb ausgestattet ist, bei dem der äußere Stelltrieb zwei Stellelemente zur unabhängigen Steuerung der Rotation und der Translation der inneren Einheit aufweist,
- Fig. 5: einen abschnittsweisen Längsschnitt des Ausführungsbeispiels von Fig. 4 in einer axialen Endposition,
- Fig. 5a: einen stark vergrößerten Ausschnitt des Schnittes von Fig. 5 in dem dort mit einem Kreis umgrenzten Bereich zur Erläuterung der Anschläge,
- Fig. 6: einen der Darstellung von Fig. 5 entsprechenden Schnitt in der anderen axialen Verschiebestellung,
- Fig. 6a: einen entsprechenden vergrößerten Ausschnitt von Fig. 6,
- Fig. 7: einen Längsschnitt eines dritten Ausführungsbeispiels eines erfindungsgemäßen Magnettriebes mit mehreren gleichsinnig gegenüberstehenden Polen von Magneten zur Zentrierung der inneren Einheit,
- Fig. 8: einen abschnittsweisen Längsschnitt eines vierten Ausführungsbeispiels eines erfindungsgemäßen Magnettriebes mit einem Haltemechanismus in den unterschiedlichen axialen Verschiebepositionen,
- Fig. 8a: eine vergrößerte Darstellung des mit einem Kreis umgrenzten Bereiches in Fig. 8,
- Fig. 9: eine der Fig. 8 vergleichbare Schnittdarstellung in einer entgegengesetzten axialen Verschiebeposition, und
- Fig. 9a: eine vergrößerte Darstellung des in Fig. 9 mit einem Kreis umgrenzten Bereiches.

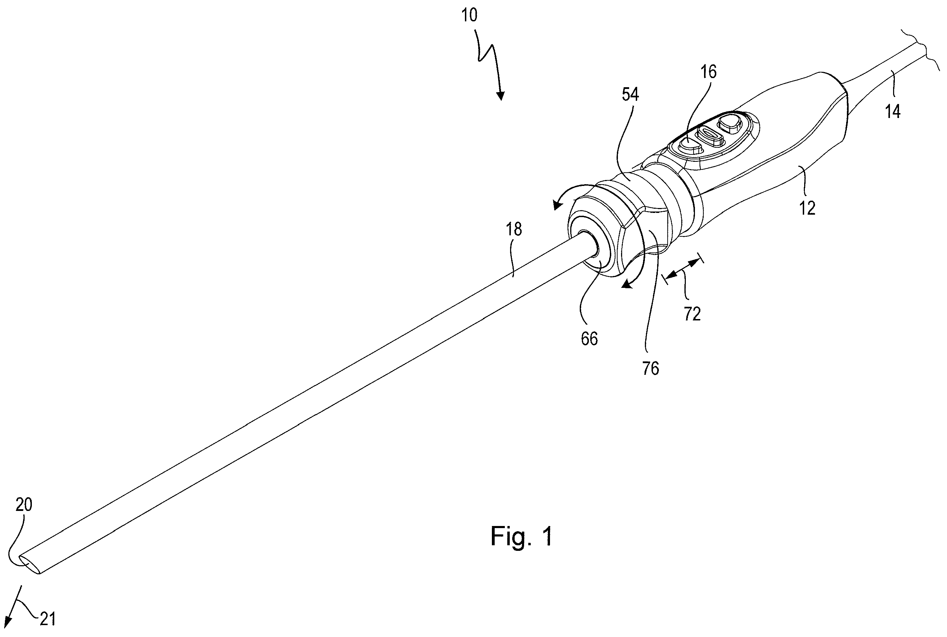

Ein in den Fig. 1 bis 3 dargestelltes erstes Ausführungsbeispiel eines medizinischen Instrumentes ist in seiner Gesamtheit mit der Bezugsziffer 10 bezeichnet.

Das medizinische Instrument 10 im gezeigten Ausführungsbeispiel ist ein Videoendoskop.

Das medizinische Instrument 10 weist proximalseitig einen Griff 12 auf, von dessen proximalem Ende ein Kabel 14 abführt. Bedienknöpfe 16 an der Außenseite des Griffes 12 dienen dazu, um gewisse Funktionen des medizinischen Instrumentes 10 zu steuern.

Distalseitig ist der Griff 12 mit einem Außenschaft 18 verbunden, an dessen distalem Endbereich ein Objektiv 20 angeordnet ist. Ein Abschlussfenster 26 bildet einen hermetisch dichten distalseitigen Abschluss des Außenschaftes 18.

Das Objektiv 20 ist so ausgebildet, dass es einen sog. Schrägblick erlaubt, wie das in Fig. 1 durch einen Pfeil 21 angedeutet ist. Das bedeutet, die Blickrichtung ist nicht geradeaus, sondern ist um die Mittellängsachse des Außenschaftes 18 um ca. 30° abgelenkt.

Im rohrförmigen Außenschaft 18 ist ein Innenschaft 22 aufgenommen.

In dem hier nicht näher bezeichneten Raum zwischen der Außenseite des Innenschaftes 22 und der Innenseite des Außenschaftes 18 sind, wie das aus dem Endoskopbau bekannt ist, Lichtleiter 24 angeordnet, die Beleuchtungslicht bis zum Abschlussfenster 26 führen.

Im Innenschaft 22 ist ein sog. Sensorschaft 28 aufgenommen, der im Innern an seinem distalen Ende einen optischen Sensor 30 trägt.

Der optische Sensor 30 in dem dargestellten Ausführungsbeispiel ist eine Videokamera mit einem CCD-Chip, der das vom Objektiv 20 kommende Bild in ein elektrisches Signal umsetzt.

Der optische Sensor 30 ist mit zumindest einer Zuleitung 32 verbunden, die durch den Sensorschaft 28 bis in das Innere eines Gehäuses 34 des Griffes 12 geführt wird.

Eine Lichtleiterzuführung 38 führt hier noch gebündelt die Lichtleiter 24 in den zuvor beschriebenen Zwischenraum zwischen Außenschaft 18 und Innenschaft 22.

In dem Gehäuse 34 ist ein Innengehäuse 36 vorhanden, das hermetisch dicht sowohl mit dem Außenschaft 18 als auch mit dem Innenschaft 22 verbunden ist.

Der Sensorschaft 28 ist um seine Längsachse drehbar im Innenschaft 22 aufgenommen. Der Sensorschaft 28 ist sowohl im Innenschaft 22 als auch im Innengehäuse 36 drehbar und axial verschiebbar gelagert. Eine Hülse 42 ist im Innern des Sensorschaftes 38 aufgenommen und trägt an ihrer Außenseite umfänglich verteilt mehrere innere Magnete 44. Dazu weist die Hülse 42 an ihrer Außenseite entsprechende Aussparungen 46 auf, in denen die Magnete 44 fest angeordnet sind.

Die Hülse 42 ist mit dem Sensorschaft 28 drehfest verbunden, d.h. die Einheit aus Sensorschaft 28, Hülse 22 und innere Magnete 44 dreht sich insgesamt.

Dieser Zusammenbau ist nicht nur drehbar, sondern in einem bestimmten Ausmaß auch axial hin und her bewegbar.

Das Ausmaß dieser Bewegung wird durch zwei Anschläge 48 und 50 bestimmt, die gegenüberliegende Flanken einer Aussparung 46 des Innengehäuses 36 darstellen, zwischen denen ein äußerer Ringflansch 52, der mit der Hülse 42 verbunden ist, laufen kann.

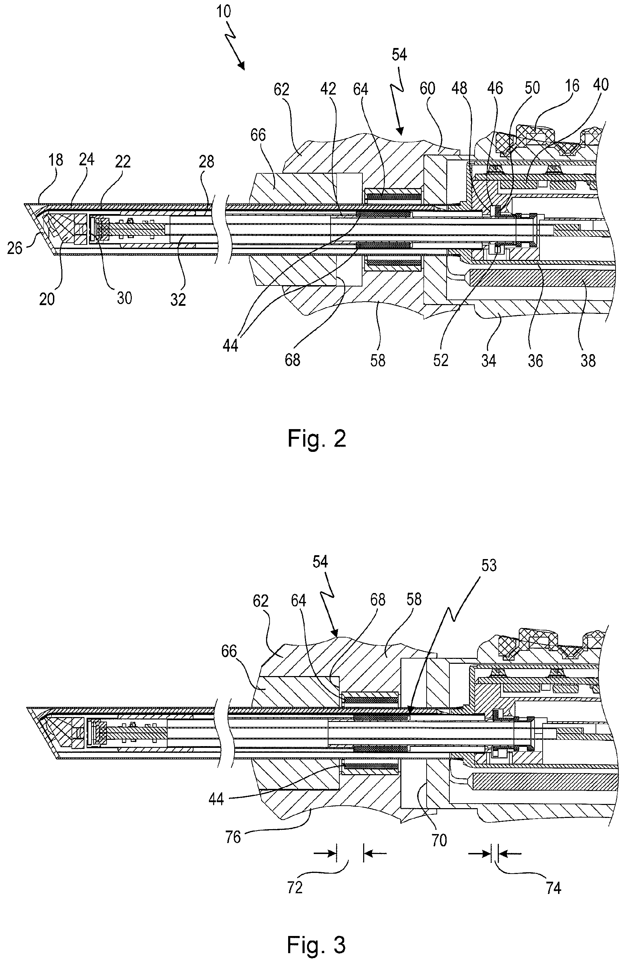

Dieser axiale Verschiebeweg 74 ist in Fig. 3 dargestellt, dieser ist relativ kurz und liegt etwa im Bereich von mehreren Zehntelmillimetern.

Der Zusammenbau aus Außenschaft 18, Innenschaft 22, Sensorschaft 28, Hülse 42, inneren Magneten 44 und Innengehäuse 36 stellt eine zur Außenseite hin hermetisch abgeschlossene innere Einheit 53 dar.

Die axiale Verschiebbarkeit des Sensorschaftes 28 dient dazu, um eine Fokussierung bzw. Scharfstellung des von dem Objektiv 12 auf den optischen Sensor 30 fallenden Bildes zu bewerkstelligen.

Die Drehbarkeit des Sensorschaftes 28 hat den Sinn, das durch den optischen Sensor 30 erfasste Bild in eine bestimmte Ausrichtung zu bringen, meist in eine Nord-Süd-Ausrichtung, nachdem das medizinische Instrument 10 um seine Längsachse gedreht worden ist.

Nimmt man beispielsweise einmal die in Fig. 1 dargestellte Ausrichtung, so erscheint ein Bild, das in Richtung des Pfeils 21 liegt.

Wird das medizinische Instrument 10 gedreht, bewegt sich der Pfeil 21 auf der Außenseite eines Kegels, nach einer Drehbewegung um 180° würde man auf dem entsprechend hier nicht dargestellten Monitor ein Bild sehen, das gegenüber dem ursprünglichen Bild auf dem Kopf steht.

Es ist aber von vielen Ärzten und Handhabungspersonen gewünscht, dass das Bild in die gewohnte Nord-Süd-Ausrichtung aufgerichtet wird, wobei dies dadurch geschehen kann, dass der Sensorschaft 28 um 180° gedreht wird.

Hierzu ist erfindungsgemäß vorgesehen, einen äußeren Stelltrieb 54 bereitzustellen, der die innere Einheit 53 bzw. deren innere Magnete 44 bewegt.

Dazu weist der äußere Stelltrieb 54 ein Stellelement 56 in Form eines Stellrades 58 auf, das den Außenschaft 18 am distalen Ende des Gehäuses 34 vollständig umgibt.

Das Stellrad 58 ist axial hin und her verschiebbar, wie das in Fig. 1 durch einen Doppelpfeil 72 dargestellt ist, somit stellt dies den axialen Verschiebeweg 74 des äußeren Stelltriebes 54 dar.

Zur Führung des Stellrades 58 weist dieses einen proximalen Ringflansch 60 auf, der das distale Ende des Gehäuses 34 übergreift. An der gegenüberliegenden Seite springt ein distaler Ringflansch 62 vor, der einen auf die Außenseite des Außenschaftes 18 befestigten Führungsring 66 übergreift.

An seiner Innenseite, in geringfügigem radialen Abstand zur Außenseite des Außenschaftes 18 liegend, sind umfänglich mehrere äußere Magnete 64 angeordnet, die der Anzahl und in ihrer Länge und Positionierung jeweils den inneren Magneten 44 entsprechen bzw. gegenüberliegen.

Wird das Stellrad 58 gedreht, werden durch die Magnetkopplung zwischen den äußeren Magneten 64 und den gegenpoligen inneren Magneten 44 die inneren Magnete 44 der Bewegung der äußeren Magnete 64 folgen.

Dadurch dreht sich dann der Sensorschaft 48, damit auch der optische Sensor 30, so dass dadurch das von diesem erfasste Bild wie zuvor beschrieben aufgerichtet werden kann. Die Drehbarkeit des Stellrades 58 beträgt mindestens 180°, im dargestellten Ausführungsbeispiel ist der Drehwinkel sogar größer.

Da ja der Sensorschaft 28 entsprechende Zu- bzw. Ableitungen 32 aufweist, die zu einer Elektronik 40 im Innern des Gehäuses 34 führen, wird dafür Sorge getragen, dass das Stellrad 58 nicht beliebig weit und schon gar nicht über 360° hinaus gedreht werden kann, ansonsten würden sich ja diese Leitungen verdrillen.

Es reicht ja für die Aufrichtung des Bildes eine Verdrehbarkeit von mindestens 180° aus, denn man kann dann ein solches auf den Kopf stehendes Bild ja entweder durch eine Drehung im Uhrzeigersinn oder entgegen dem Uhrzeigersinn entsprechend aufrichten.

Wird das Stellrad 58 axial hin und her bewegt, so kann dies um einen Verschiebeweg 72 verschoben werden, wie er in Fig. 1 und 3 dargestellt ist.

In Fig. 2 ist das Stellrad 58 in seiner maximal nach proximal verschobenen Stellung dargestellt, dabei liegt die proximale Ringfläche des Stellrades 58 an einem Anschlag 70 (siehe Fig. 3) am Gehäuse 34 an.

Der Übergang von Fig. 2 zu Fig. 3 zeigt den maximalen axialen Verschiebeweg 72, hier von proximal nach distal. Wie aus Fig. 3 ersichtlich, liegt dann die entsprechende distale Stirnfläche des Stellrades 58 an einem Anschlag 68 am Führungsring 66 an.

Aus Fig. 3 ist zu entnehmen, dass der axiale Verschiebeweg 72 des Stellrades 58 wesentlich länger ist als der durch die Anschläge 48 und 50 bestimmte Verschiebeweg 74 des Sensorschaftes 28.

Aus dem Übergang von Fig. 2 zu Fig. 3 ist auch zu erkennen, dass in der in Fig. 2 dargestellten Position die äußeren Magnete 64 etwas über die inneren Magnete 44 axial hinausgeschoben sind bzw. diese teilweise überfahren haben.

Entsprechendes gilt für die andere axiale Verschiebeendposition, wie sie in Fig. 3 dargestellt ist, hier haben die äußeren Magnete 64 die inneren Magnete 44 in distaler Richtung etwas überfahren.

Diese konstruktive Ausgestaltung führt nun dazu, dass die Magnetkopplung zwischen den äußeren Magneten 64 und den inneren Magneten 44 bewirkt, dass die inneren Magnete 44 jeweils dazu tendieren, weiter verschoben zu werden, was aber durch die Anschläge 48 bzw. 50 gehindert ist.

Die Magnetkopplung bewirkt somit, dass der zwischen den Anschlägen 48 und 50 sich hin und her bewegende äußere Ringflansch 52 in den Endpositionen an diese Anschläge angedrückt wird.

Dies resultiert in einer gefederten Lagerung des äußeren Ringflansches 42 und somit der gesamten inneren Einheit 53 in diesen Endpositionen.

Dies resultiert gleichzeitig in einer festen Positionierung der inneren Einheit 53 in diesen Extrempositionen. Beispielsweise wird der optische Sensor 30 in zwei Extremstellungen, nämlich "Naheinstellung" und "Ferneinstellung", gehalten.

Soll beispielsweise mit dem medizinischen Instrument eine Harnblase inspiziert werden, kann in dem Zustand "Naheinstellung" genau die Blaseninnenwand mit hoher Sehschärfe inspiziert werden. Soll ein Gesamtbild der Blaseninnenseite vermittelt werden, kann das distale Ende des Außenschaftes 18 etwa in der Mitte der Blase positioniert werden, die Einstellung "Fernsicht" eingestellt werden und somit ein scharfer Rundumblick der gesamten Innenseite oder Innenwand der Harnblase erhalten werden.

Die zuvor erwähnte Federung sorgt auch dafür, dass schlag- oder schockartige mechanische Einwirkungen auf das medizinische Instrument 10 in axialer Richtung nicht dafür sorgen, dass sich der Sensorschaft aus einer bestimmten axialen Verschiebeposition ungewollt verschiebt.

Wird das Stellrad 58 aus der in Fig. 2 dargestellten Position nach distal, also in Fig. 2 nach links bewegt, bewegt sich zunächst der Sensorschaft 28 nicht, erst wenn die inneren Magnete 44 und äußeren Magnete 64 etwa deckungsgleich übereinander liegen, hebt der äußere Ringflansch 52 von dem Anschlag 50 ab und der Sensorschaft 28 wird um den Verschiebeweg 74 nach distal verschoben. Diese Verschiebebewegung endet in dem Moment, in dem der äußere Ringflansch 52 auf den Anschlag 46 trifft. Das äußere Stellrad 58 kann aber noch weiter axial bewegt werden, bis die in Fig. 3 dargestellte Stellung erreicht ist, so dass auch in dieser Position dann wieder der "Federungseffekt" erzielt wird.

Bei dem zuvor in Zusammenhang mit Fig. 1 bis 3 beschriebenen äußeren Stelltrieb 54 handelt es sich um einen Stelltrieb, der nur ein einziges Stellelement 56 in Form des Stellrades 58 aufweist. Das heißt, mit dem Stellrad 58 werden beide Bewegungen, also sowohl die Rotation als auch die Translation gesteuert.

Zur Erleichterung der Handhabung und auch zur Orientierung ist an der Außenseite des Stellrades 58 eine Griffmulde 76 vorhanden, über die beispielsweise ein Daumen einer Bedienungsperson eingelegt werden kann und darüber die Bewegungen steuert.

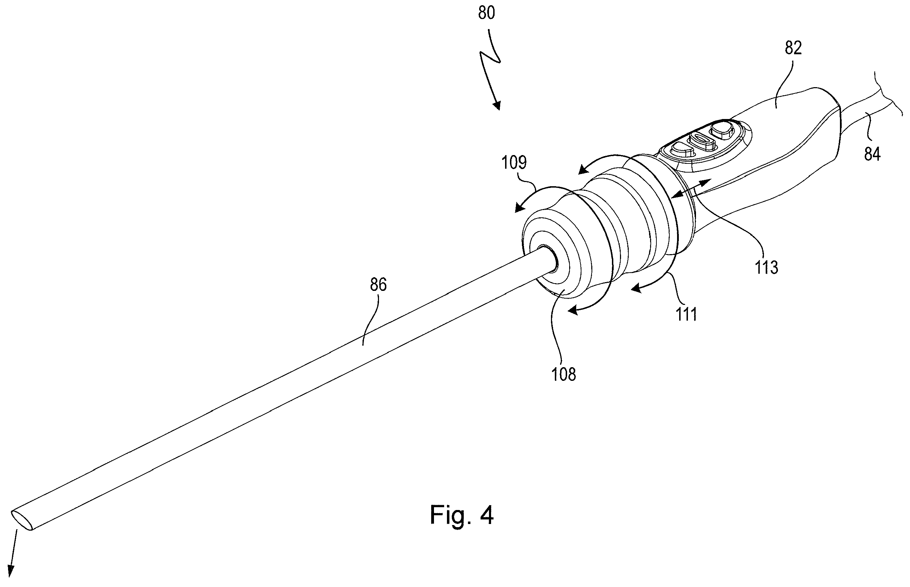

Bei dem nachfolgend in Zusammenhang mit den Figuren 4 bis 6a beschriebenen Ausführungsbeispiel ist der äußere Stelltrieb so ausgestaltet, dass er zwei Stellelemente aufweist, wobei eines für die Steuerung der Rotation und das andere für die Steuerung der Translation herangezogen wird.

Das in Fig. 4 dargestellte zweite Ausführungsbeispiel eines medizinischen Instrumentes ist in seiner Gesamtheit mit der Bezugsziffer 80 bezeichnet.

Das medizinische Instrument 80 ist sehr ähnlich wie das medizinische Instrument 10 aufgebaut, so dass nachfolgend im Wesentlichen die Unterschiede beschrieben werden.

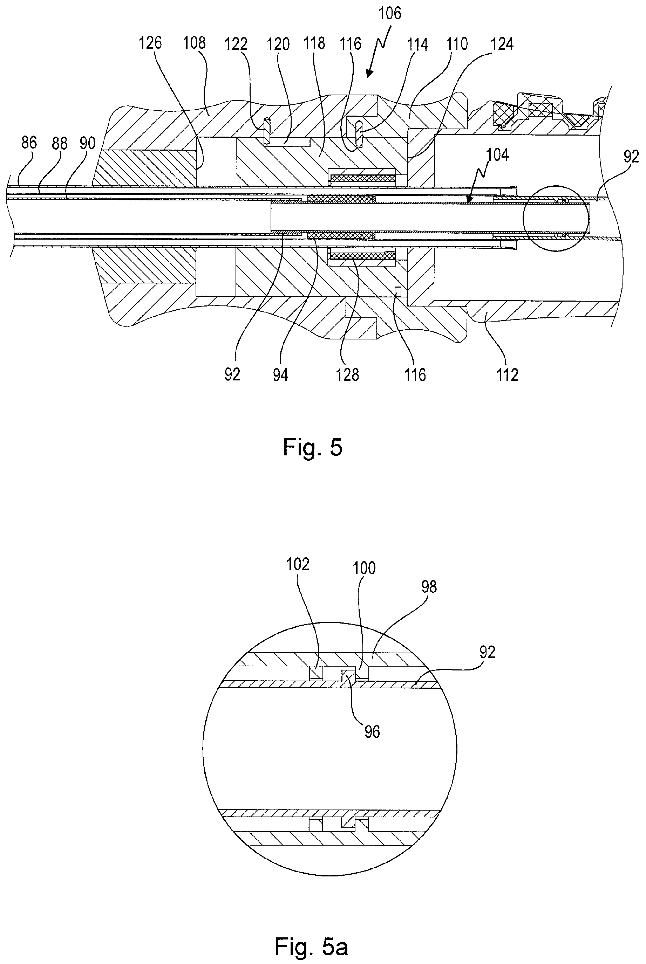

Demzufolge weist, wie das insbesondere aus den Figuren 4 und 5 ersichtlich ist, das medizinische Instrument 80 einen Griff 82 auf, von dem proximalseitig ein Kabel 84 vorsteht.

Der Griff 82 ist mit einem Außenschaft 86 verbunden, in dem ein Innenschaft 88 angeordnet ist, in diesem Inneren wiederum der drehbare und axial verschiebbare Sensorschaft 90 aufgenommen ist. Auch hier ist wieder der Sensorschaft 90 mit einer Hülse 92 verbunden, die an ihrer Außenseite die inneren Magnete 94 trägt.

Die Hülse 92 erstreckt sich bei diesem Ausführungsbeispiel weit in den Innenraum des Griffes 82 hinein und weist einen äußeren Ringflansch 96 auf, wie er insbesondere aus der vergrößerten Darstellung von Fig. 5a ersichtlich ist. Dieses innere Ende der Hülse 92 ist von einem Rohr 98 umgeben, von dessen Innenseite ein innerer Ringflansch 100 sowie ein weiterer innerer Ringflansch 102 vorsteht.

Bei der Montage wird das Rohr 98 so über den Endbereich der Hülse 92 gebracht, dass der äußere Ringflansch 96 der Hülse 92 zwischen den inneren Ringflanschen 100 und 102 zum Liegen kommt.

Diese Ausgestaltung stellt die Begrenzung des axialen Verschiebeweges der Hülse 92 dar, wie das schon zuvor in Zusammenhang mit dem ersten Ausführungsbeispiel beschrieben wurde. Auch hier bildet wieder der axial verschiebbare und drehbare Sensorschaft 90 zusammen mit der Hülse 92 und den die Hülse 92 umgebenden Schäften eine zur Außenseite hin dichtend abgeschlossene innere Einheit 104.

Wie bereits erwähnt, ist der äußere Stelltrieb 106 so aufgebaut, dass er ein erstes Stellelement 108 und ein zweites Stellelement 110 aufweist.

Das erste Stellelement 108 besteht wieder in Form eines hier nicht näher bezeichneten Stellrades und dient dazu, um die Drehbewegung der inneren Einheit 104 zu bewerkstelligen. Dazu ist das erste Stellelement 108 drehbar, wie das durch einen Pfeil 109 angedeutet ist. Aus der Schnittdarstellung von Fig. 5 ist zu erkennen, dass das erste Stellelement 108 distalseitig auf einem hier nicht näher bezeichneten Führungsring läuft, der proximalseitig eine Anschlag 126 aufweist.

Das erste Stellelement 108 sitzt ferner auf einem hülsenartigen Körper 118, der auf seiner Außenseite eine axiale Nut 120 aufweist, in die ein von der Innenseite des ersten Stellelements 108 radial vorspringender Mitnehmerstift 122 hineinragt.

Der hülsenartige Körper 118 trägt an seiner Innenseite die entsprechenden äußeren Magnete 128, die mit den inneren Magneten 94 an der Hülse 92 magnetisch koppeln.

Das erste Stellelement 108 übergreift proximalseitig teilweise ein zweites Stellelement 110, das drehbar am distalen Ende des Griffes 82 angebracht ist, wie das insbesondere aus Fig. 5 ersichtlich ist.

Das zweite Stellelement 110 ist ebenfalls drehbar, wie das in Fig. 4 durch einen Pfeil 111 dargestellt ist, allerdings wird diese Drehbewegung in eine axiale Bewegung des hülsenartigen Körpers 118 umgesetzt, der die äußeren Magnete 128 trägt.

Diese Bewegung ist in Fig. 4 durch den Pfeil 113 angedeutet.

Um diese Drehbewegung des zweiten Stellelementes 110 in eine axiale Verschiebung des hülsenartigen Körpers 118 umzusetzen, steht von der Innenseite des zweiten Stellelementes 110 radial ein Stift 114 vor, der in eine in die Außenseite des hülsenartigen Körpers 118 eingeschnittene Wendelnut 116 eingreift.

Wird somit das zweite Stellelement 110 gedreht, wird diese Drehbewegung über den Stift 114 und die Wendelnut 116 in eine axiale Verschiebung des hülsenartigen Körpers 118 umgesetzt.

Das erste Stellelement 108 bleibt von dieser Verschiebung unberührt, denn die axiale Nut 120 an der Außenseite des hülsenartigen Körpers 118 und der Mitnehmerstift 122 erlauben diese axiale Verschiebung relativ zum ersten Stellelement 108.

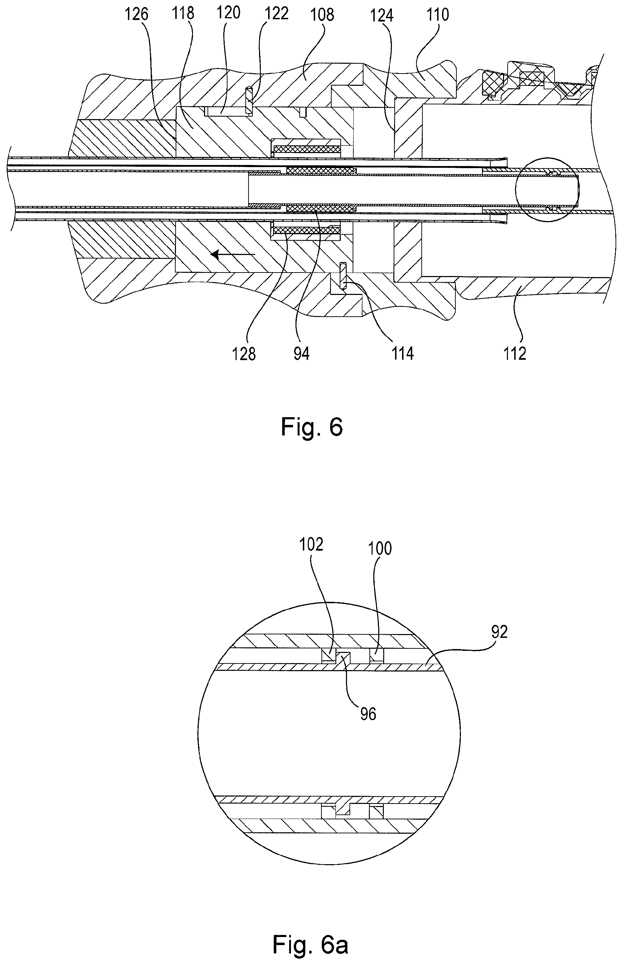

Diese axiale Verschiebung und somit die axiale Verschiebung des Sensorschaftes 90 ist beispielsweise aus dem Übergang von Fig. 5 zu Fig. 6 zu erkennen.

In der Darstellung von Fig. 5 ist zu erkennen, dass der hülsenartige Körper 118 an einem distalen Anschlag 124 des Gehäuses 112 des Griffes 82 anliegt. Diese Situation stellt den maximalen axialen Verschiebeweg des hülsenartigen Körpers 118 nach proximal dar.

Auch hier haben die äußeren Magnete 128 etwas die inneren Magnete 94 überfahren und sorgen für den zuvor beschriebenen Federungseffekt.

Dabei liegt der äußere Ringflansch 96 am inneren Ringflansch 100 an, wie das aus der vergrößerten Darstellung von Fig. 5a ersichtlich ist.

Wird nunmehr das zweite Stellelement 110 gedreht, wird die Drehbewegung in die axiale Verschiebbewegung des hülsenartigen Körpers 118 umgesetzt und dieser verschiebt sich nach proximal, in der Darstellung von Fig. 5 nach links.

Das Ende dieser Bewegung ist in Fig. 6 dargestellt, d.h., das distale Ende des hülsenartigen Körpers 118 ist auf den Anschlag 126 gelaufen.

Wie insbesondere aus Fig. 6 zu erkennen ist, haben auch hier wieder die äußeren Magnete 128 die inneren Magnete 94 etwas überlaufen und pressen bzw. drücken den äußeren Ringflansch 96 gegen den inneren Ringflansch 102 (siehe Fig. 6a).

Auch hier ist wieder der axiale Verschiebeweg des hülsenartigen Körpers 118 wesentlich länger als der axiale Verschiebeweg des Sensorschaftes 90. Der axiale Verschiebeweg des hülsenartigen Körpers 118 beträgt beispielsweise 10 mm, wohingegen der axiale Verschiebeweg des Sensorschaftes 90 zwischen den beiden Anschlägen 100 nur 0,2 mm beträgt.

Der Sensorschaft 90 trägt dieselben Bauelemente wie zuvor beschrieben, also einen optischen Sensor, und der Außenschaft 86 trägt ein entsprechendes Objektiv mit Seitblick.

Durch Drehen des ersten Stellelementes 108 wird der hülsenartige Körper 118 gedreht, was durch den Mitnehmerstift 122 verursacht wird, der in der axialen Nut 120 sitzt.

Das erste Stellelement 108 kann sich dabei um das zweite Stellelement 110 drehen, das bei dieser Drehbewegung nicht mitbewegt wird, denn ansonsten würde sich der Sensorschaft 90 axial verschieben.

Diese Ausgestaltung wird immer dann gewählt, wenn es die Handhabungspersonen bzw. die Ärzte wünschen, beide Bewegungen, also sowohl Rotation als auch Translation, durch drehbare Stellelemente zu bewirken. In diesem Fall muss man dann entsprechende Hinweise oder Schulungen durchführen, damit die Handhabungsperson weiß, welcher dieser beiden Drehtriebe die Rotation und welcher die Translation verursacht. Dies kann man beispielsweise durch entsprechende optische Hinweise, wie unterschiedliche Farbgebung oder durch auf der Außenseite angebrachte Markierungen, wie Pfeile, bewerkstelligen.

Aus der Schnittdarstellung bspw. von Fig. 6 ist zu erkennen, dass man das zweite Stellelement 110 auch so mit dem hülsenartigen Körper 118 verbinden könnte, dass damit unmittelbar die axiale Verschiebbarkeit erfolgt. Allerdings müsste sich dann das zweite Stellelement 110 axial vom ersten Stellelement 108 wegbewegen können, um diese Verschiebebewegung zu erlauben. Das würde nunmehr verursachen, dass in den dadurch gebildeten Spalt Verschmutzungen eintreten können, die die axiale Verschiebung der beiden Stellelemente zueinander hindern oder blockieren.

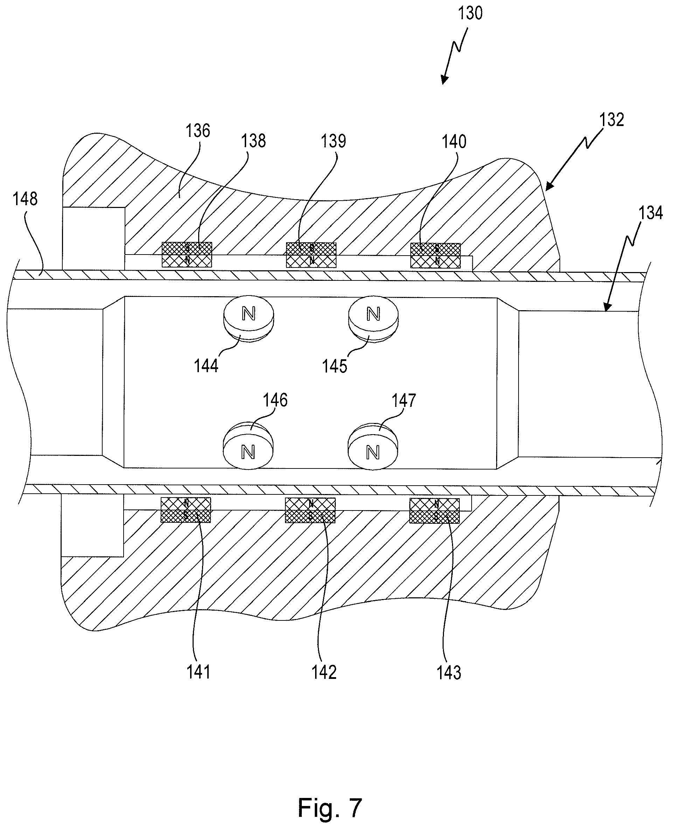

In Fig. 7 ist ein drittes Ausführungsbeispiel eines Magnettriebes 130 dargestellt, bei dem eine mögliche Polung der Magnete näher erläutert wird.

Auch hier ist wieder ein äußerer Stelltrieb 132 vorhanden, der dazu vorgesehen ist, eine in sich gegenüber der Außenseite hermetisch abgeschlossene innere Einheit 134 zu bewegen. Die innere in sich abgeschlossene Einheit 134 kann beliebig ausgestaltet sein, sie soll um ein bestimmtes Maß axial verschoben und auch gedreht werden können.

Der äußere Stelltrieb 132 weist dazu ein Stellrad 136 auf, das an einer an seiner Innenseite mit einer Vielzahl von umfänglich und auch axial verteilten Magneten 138 bis 143 versehen ist. In der Schnittdarstellung von Fig. 7 sind nur die gegenüberliegenden Magnete 138, 139, 140 bzw. 141, 142, 143 zu sehen, es sind umfänglich zwischen den Magneten 138 und 141 weitere zwei Magnete enthalten.

Die Außenseite der inneren Einheit 134 ist ebenfalls mit mehreren umfänglich und axial voneinander beabstandeten Magneten 144, 145, 146, 147 versehen, die so gepolt sind, dass deren äußere Pole, hier beispielsweise der N-Pol, dem entsprechenden N-Pol der äußeren Magnete 138 - 143 des Stellrades 136 gegenüberstehen. Auch hier sind an der Außenseite der inneren Einheit 134 entsprechend weitere Magnete vorhanden.

Der äußere Stelltrieb 132 ist drehbar und axial verschiebbar auf einem Schaft 148 montiert.

In dessen Innenraum ist die schaftartige innere Einheit 134 eingeschoben.

Durch die Abstoßung der Magnete findet eine Selbstzentrierung der inneren Einheit 134 im Inneren des Schaftes 148 statt. Dies erlaubt einen besonderen ruhigen zentrischen Lauf der inneren Einheit 134 im Schaft 148. Wird der äußere Stelltrieb 132 gedreht, ist die Magnetkopplung so, dass die Abstoßung zwischen den N-Polen bewirkt, dass sich auch die innere Einheit 134 dreht.

Wird der äußere Stelltrieb 132 axial verschoben, verursacht die abstoßende Magnetkopplung entsprechend eine lineare bzw. axiale Verschiebung der inneren Einheit 134.

Beim Montieren der inneren Einheit 134 müssen deren N-Pole unter Überwindung der Abstoßungskraft der N-Pole des äußeren Stelltriebes 132 beispielsweise in die in Fig. 7 dargestellte Position eingeschoben werden.

Somit wird nicht nur eine Zentrierung bewirkt, sondern auch eine Halterung des jeweiligen axialen Verschiebeweges bewirkt.

So wird beispielsweise der N-Pol 144 immer in einer Position gehalten, in der er etwa mittig zwischen den N-Polen 138 bzw. 139 des äußeren Stelltriebes 132 liegt, konkret, d.h. in dreidimensionaler Betrachtung, mittig im Rechteck zwischen den N-Polen 138 bzw. 139 und ihren jeweils tangential benachbarten N-Polen.

Dies eröffnet nunmehr auch die Möglichkeit, den äußeren Stelltrieb 132 in bestimmten kleinen Inkrementen zu verschieben, denen die innere Einheit 134 in entsprechenden Schritten folgt.

In anderen Worten ausgedrückt, kann hier die innere Einheit 134 in jedem beliebigen axialen Verschiebeweg durch die Gegenpoligkeit gehalten werden und ebenso in jeder tangentialen Position, d.h. gegen Verdrehen. Erst wenn die zuvor beschriebene starke Kraft zur Überwindung der Abstoßung überwunden wird, beispielsweise beim Montieren, kann der N-Pol 144 beispielsweise nach links über den N-Pol 138 hinausgeschoben werden.

Da solch starke Kräfte aber im Betrieb nicht einwirken, kann hier eine sehr fein justierbare axiale Verschiebung durchgeführt werden.

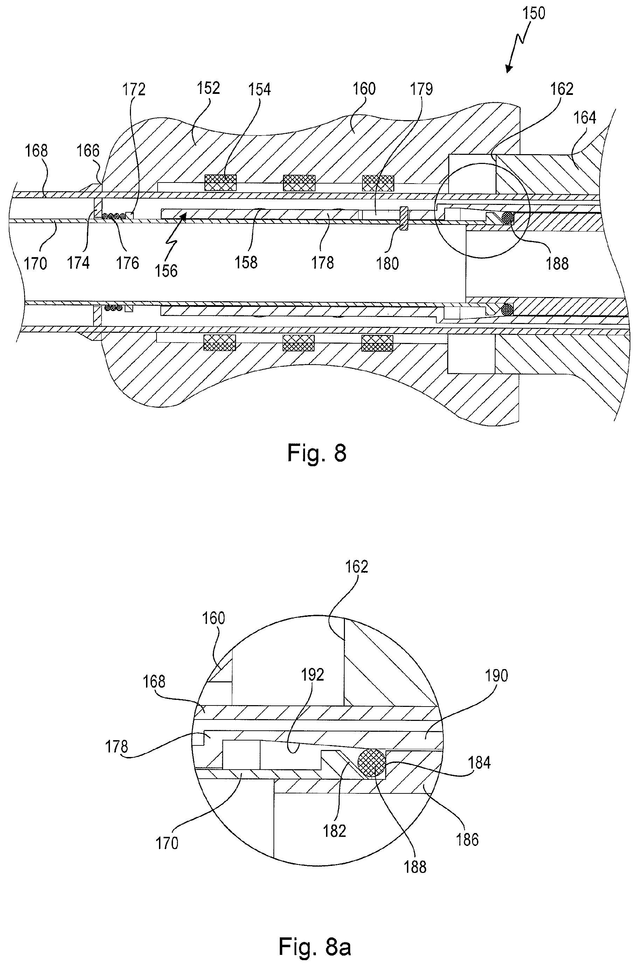

Bei dem in den Fig. 8 bis 9a gezeigten vierten Ausführungsbeispiel sind konstruktive Maßnahmen getroffen, um einen solchen Magnettrieb durch einen Haltemechanismus zu halten, wobei der Haltemechanismus zugleich die Möglichkeit eröffnet, eine Übersetzung (oder eine Untersetzung) einer axialen Verschiebung des äußeren Stelltriebes in andere, vorzugsweise geringere, Verschiebung der inneren Einheit, gegebenenfalls auch in eine mehr oder weniger gleich lange Verschiebung der inneren Einheit umzusetzen.

Das in den Fig. 8 bis 9a gezeigte weitere Ausführungsbeispiel eines medizinischen Instrumentes 150 ist lediglich im Bereich des äußeren Stelltriebes 152 dargestellt.

Wie zuvor in dem Ausführungsbeispiel im Zusammenhang mit Fig. 7 beschrieben, sind an der Innenseite des äußeren Stelltriebes 152 umfänglich und axial verteilt mehrere Magnete 154 angeordnet. Auch hier sitzt der äußere Stelltrieb auf einem Schaft 168.

Im Schaft 168 ist ein Sensorschaft 170 angeordnet, auf den eine Außenhülse 178 aufgeschoben ist, an deren Außenseite umfänglich und axial verteilt entsprechende Magnete 158 angeordnet sind.

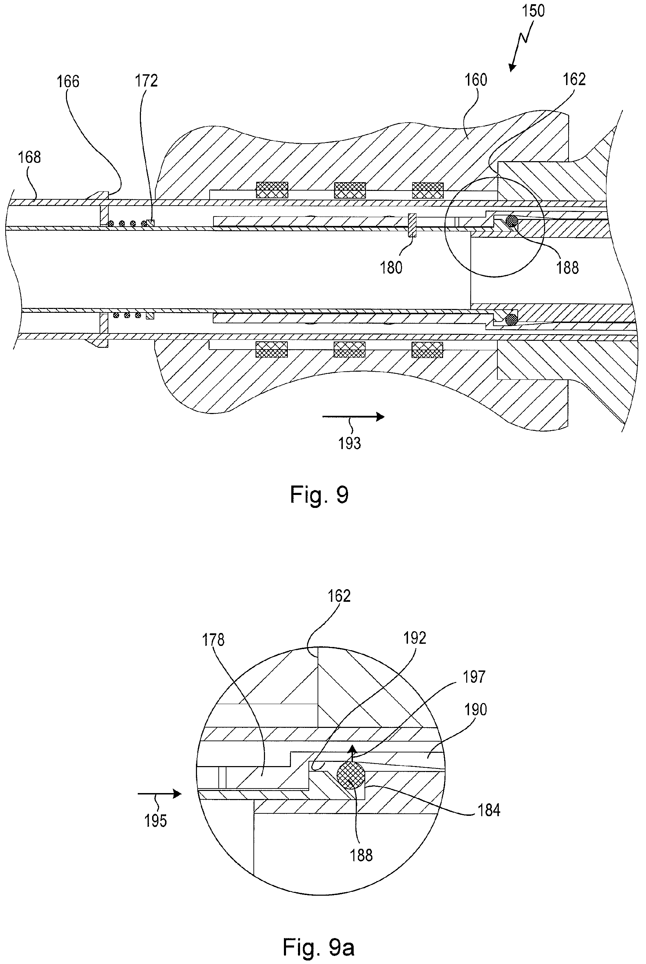

Der äußere Stelltrieb 152, wie das vom Übergang von Fig. 8 zu Fig. 9 ersichtlich ist, ist zwischen einer axialen Position, bei der dieser an einem Anschlag 166 an der Außenseite des Schaftes 180 anliegt und einem Anschlag 162 am Gehäuse 164 des Instrumentes hin und her bewegbar. Aufgrund der zuvor beschriebenen gleichpoligen Ausrichtung wird dann auch der Sensorschaft 170 entsprechend axial verschoben. Hierbei ist alternativ allerdings auch eine gegenpolige Ausrichtung möglich.

Wie insbesondere aus der stark vergrößerten Darstellung von Fig. 8a zu erkennen ist, ist das proximalseitige Ende des Sensorschaftes 170 mit einer Schräge 182 versehen.

Auf der Außenseite des Sensorschaftes ist ja die Hülse 178 aufgebracht, die die Magnete 158 trägt.

Aus Fig. 8a ist nun zu erkennen, dass die Hülse 178 eine nach proximal gerichtete Verlängerung 190 aufweist, die die Schräge 182 überragt und sich über ein Innenrohr 186 weiter fortsetzt.

Das Innenrohr 186 weist eine Schulter 184 auf, die sich radial erstreckt und die der Schräge 182 genau gegenübersteht.

Zwischen der Schulter 184 und der Schräge 182 befindet sich eine Kugel 188.

Es sind hier umfänglich verteilt mehrere solche Kugeln 188 vorhanden.

In radialer Richtung ist die Kugel 188 zum einen an ihrer radial äußeren Seite durch das Innenrohr 186 gefangen, an ihrer radial inneren Seite durch die Innenseite der Verlängerung 190 der Außenhülse 178.

Die der Kugel 188 zugewandte innere Seite der Verlängerung 190 ist nun ebenfalls mit einer Schräge 192 versehen.

Aus der Darstellung von Fig. 8 ist zu erkennen, dass an der Außenseite des Sensorschaftes 170 ein Ringflansch 172 vorsteht und dass von der Innenseite des Schaftes 168 ein radial nach innen vorspringender Flansch 174 vorsteht. Dieser Flansch 174 liegt distalseitig zum Flansch 172.

Zwischen diesen Flanschen 172 und 174 ist eine Druckfeder 176 gelagert, die so vorgespannt ist, dass sie dazu neigt, den Sensorschaft 170 nach proximal, also in der Fig. 8 nach rechts zu drücken. Ein Mitnehmerstift 180 sorgt für eine drehschlüssige Verbindung zwischen Hülse 178 und Sensorschaft 170. Das heißt, wird der äußere Stelltrieb 152 gedreht, drehen sich sowohl die Hülse 178 als auch der Sensorschaft 170.

Eine entsprechende Öffnung 179 in der Hülse 178, in die der Mitnehmerstift 180 hineinragt, erlaubt allerdings eine relative axiale Verschiebung zwischen Hülse 178 und Sensorschaft 170.

In den Fig. 8 und 8a ist eine Situation dargestellt, bei der der äußere Stelltrieb 152 sich in einer axialen Endposition befindet, nämlich der maximal nach distal verschobenen Position.

Wird nunmehr der äußere Stelltrieb 152 nach proximal verschoben, also in der zeichnerischen Darstellung nach rechts, nehmen die Magnete 154 die Magnete 158 mit.

Dadurch verschiebt sich die Verlängerung 190 über der Kugel 188 nach proximal, so dass dieser die Möglichkeit gegeben wird, sich radial nach außen zu bewegen.

Durch die Kraft der vorgespannten Feder 176 wird nunmehr auch der Sensorschaft 170 etwas nach proximal verschoben und zwar so weit, bis die Kugel 188 an der Schräge 192 liegt.

Der axiale Verschiebeweg der Hülse 178 ist dann beendet, wenn der äußere Stelltrieb 152 auf den Anschlag 162 am Gehäuse 164 trifft. Die Länge der Öffnung 179 ist dann so lang, dass dieser maximale Weg möglich ist.

Der Verschiebeweg des Sensorschaftes 170 ist dabei wesentlich geringer als der Verschiebeweg der Hülse 178 und nur so lang, wie es der radial nach innen gerichtete Verschiebeweg der Kugel 188 erlaubt.

Durch diese Ausgestaltung wird zum einen ein Art Haltemechanismus geschaffen, der das System in jedem beliebigen axialen Verschiebeweg hält. Dies dadurch, da die vorgespannte Feder 176 die Schräge 182 gegen die Kugel 188 drückt, diese aber nur soweit radial ausrücken kann, wie dies die Schräge 192 an der Unterseite der Verlängerung 190 erlaubt. In einer solchen radialen Verschiebeposition kann die Kugel aber nicht mehr radial nach innen ausweichen, denn die Schräge 182 hat die Kugel 188 entsprechend unterlaufen, wie das beispielsweise aus Fig. 9a ersichtlich ist.

Durch das Verhältnis der Steigung der Schräge 182 zur Steigung der Schräge 192, insbesondere durch Wahl der Steigung der Schräge 192 kann nun das Übersetzungsverhältnis eingestellt werden.

Beträgt die Steigung genau 45° wie im dargestellten Ausführungsbeispiel, findet eine 1:1-Umsetzung statt, d.h. um den gleichen Weg wie sich die Kugel 188 radial bewegt, bewegt sich die Schräge 182 axial.

Durch eine andere Winkelwahl kann dann ein entsprechendes Übersetzungsverhältnis ausgewählt werden.

Wäre der Winkel der Schräge beispielsweise wesentlich geringer, müsste sich die Schräge um ein Vielfaches axial vorbewegen, um die Kugel über ihren maximalen radialen Verschiebeweg zu bewegen.

Diese Ausgestaltung bildet somit neben dem Selbsthaltemechanismus auch die Möglichkeit einer äußerst fein justierbaren axialen Verschiebung des Sensorschaftes 170.

Ist der Sensorschaft 170 wie eingangs beschrieben, können somit Fokussierungen nicht nur in den Endstellungen Nahbereich und Fernbereich eingenommen, sondern auch in allen Zwischenstellungen und in diesen Zwischenstellungen gehalten werden.

## Patentansprüche

1. Medizinisches Instrument, mit einem zumindest einen Magneten (64, 128) aufweisenden äußeren Stelltrieb (54, 106, 132, 152), der mit einer im Innern des medizinischen Instrumentes (10, 80, 150) angeordneten, bewegbaren, zumindest einen Magneten (44, 94) aufweisenden inneren Einheit (53, 104, 134, 156) derart magnetisch gekoppelt ist, dass ein Bewegen des äußeren Stelltriebes eine Bewegung der inneren bewegbaren Einheit verursacht, wobei der zumindest eine Magnet (64, 128) des äußeren Stelltriebes (54, 106, 132, 152) sowohl axial verschiebbar als auch drehbar ausgebildet ist, und dass ferner die innere bewegbare Einheit (53, 104, 132, 156) sowohl axial verschiebbar als auch drehbar ausgebildet ist, und dass die magnetische Kopplung zwischen dem äußeren Stelltrieb (54, 106, 132, 152) und der inneren Einheit (53, 104, 132, 156) derart ausgebildet ist, dass eine Rotation des äußeren Stelltriebes eine Rotation der inneren Einheit bewirkt, und dass eine axiale Verschiebung des zumindest einen Magneten (64, 128) desselben Stelltriebes eine axiale Verschiebung der inneren Einheit verursacht, und wobei der axiale Verschiebeweg des äußeren Stelltriebes (152) über eine Übersetzung in einen relativ dazu gesehen kürzeren Verschiebeweg der inneren Einheit (156) umsetzbar ist,
dadurch gekennzeichtnet,
dass zwischen einer Schräge (182) der inneren Einheit (156) und einer Schräge (192) einer umgebenden Hülse (178) zumindest eine Kugel (188) angeordnet ist, die radial zwischen den Schrägen (182, 192) bewegbar ist, und dass die Steigung der Schräge (182) ein Übersetzungsverhältnis zwischen axialem Verschiebeweg des äußeren Stelltriebes (152) und einem axialen Verschiebeweg der inneren Einheit (156) bestimmt.

2. Medizinisches Instrument nach Anspruch 1, **dadurch gekennzeichnet, dass** der äußere Stelltrieb (54, 150) ein einziges Stellelement (56, 160) aufweist, das sowohl drehbar als auch axial verschiebbar ist.

3. Medizinisches Instrument nach Anspruch 1, **dadurch gekennzeichnet, dass** der äußere Stelltrieb (106) zwei Stellelemente (108, 110) aufweist, ein erstes Stellelement (108) zum Bewirken der Rotation der inneren Einheit (104) und ein zweites Stellelement (110) zum Bewirken der axialen Verschiebung der inneren Einheit (104).

4. Medizinisches Instrument nach Anspruch 3, **dadurch gekennzeichnet, dass** das zweite Stellelement (110) zum Bewirken der axialen Verschiebung der inneren Einheit (104) drehbar ausgebildet ist und mit einem hülsenartigen Körper (118) in Verbindung steht, der die Drehbewegung des zweiten Stellelementes (110) in eine axiale Verschiebung des zumindest einen Magneten (128) des äußeren Stelltriebes (106) verursacht.

5. Medizinisches Instrument nach Anspruch 3 oder 4, **dadurch gekennzeichnet, dass** das erste Stellelement (108) derart am zweiten Stellelement (110) angebracht ist, das ein Drehen des ersten Stellelementes (108) ein Drehen der inneren Einheit (104) aus jeder beliebigen axialen Verschiebestellung des äußeren Stelltriebes (106) verursacht.

6. Medizinisches Instrument nach einem der Ansprüche 1 bis 5, **dadurch gekennzeichnet, dass** die innere Einheit (53, 104, 156) zwischen zwei Anschlägen (48, 50; 100, 102) axial hin und her bewegbar ist, der axiale Verschiebeweg (72) des äußeren Stelltriebes (54) aber um ein Mehrfaches länger ist als der Verschiebeweg (74) zwischen den beiden Anschlägen (48, 50; 100, 108).

7. Medizinisches Instrument nach einem der Ansprüche 1 bis 6, **dadurch gekennzeichnet, dass** die innere bewegbare Einheit (53, 104) eine Hülse (42, 118) aufweist, die den zumindest einen Magneten (44, 94) der inneren Einheit (53, 104) trägt.

8. Medizinisches Instrument nach Anspruch 7, **dadurch gekennzeichnet, dass** der äußere Stelltrieb (54, 106, 132, 152) als Stellrad (58) ausgebildet ist, das den zumindest einen Magneten (64, 128) trägt, und dass das Stellrad (58) die Hülse (42, 92) der inneren Einheit (53, 104, 156) umgibt.

9. Medizinisches Instrument nach einem der Ansprüche 1 bis 8, **dadurch gekennzeichnet, dass** an der inneren Einheit (134, 156) umfänglich und axial verteilt mehrere Magnete (144-147; 158) angeordnet sind, und dass am äußeren Stelltrieb (132, 152) ebenfalls umfänglich und axial verteilt mehrere Magnete (138-143; 154) angeordnet sind, wobei sich gleiche Pole der inneren bzw. der äußeren Magnete gegenüberstehen.

10. Medizinisches Instrument nach einem der Ansprüche 1 bis 9, **dadurch gekennzeichnet, dass** die axiale Verschiebung der inneren Einheit (156) gegen einen Haltemechanismus erfolgt, der die innere Einheit (156) in jeder beliebigen axialen Verschiebestellung hält.

11. Medizinisches Instrument nach Anspruch 1, **dadurch gekennzeichnet, dass**, indem die Schräge (182) der inneren Einheit mit Federkraft beaufschlagbar ist, die zumindest eine Kugel (188) gegen eine Schulter (184) drückbar ist.

## Claims

1. Medical instrument having an outer actuating drive (54, 106, 132, 152) comprising at least one magnet (64, 128) which is magnetically coupled in such a way with an inner unit (53, 104, 134, 156) comprising at least one magnet (44, 94) and being arranged within an inner space of the medical instrument (10, 80, 150), that a movement of the outer actuating drive causes a movement of the inner movable unit, wherein the at least one magnet (64, 128) of the outer actuating drive (54, 106, 132, 152) is axially movable and rotatable, and in that the inner movable unit (104, 132, 156) is axially movable and rotatable, and wherein the magnetic coupling between the outer actuating drive (54, 106, 132, 152) and the inner unit (53, 104, 132, 150) is designed in that a rotation of the outer actuating drive causes a rotation of the inner unit, and in that an axial displacement of the at least one magnet (54, 128) of the same actuating drive causes an axial displacement of the inner unit, and wherein the axial displacement distance of the outer actuating drive (152) is transmittable via a transmission into a shorter displacement distance of the inner unit (156) relative thereto, **characterized in that** at least one ball (188) is arranged between a bevel (182) of the inner unit (156) and a bevel (192) of a surrounding bushing (178), which ball is radially movable between the bevels (182, 192), and **in that** the inclination of the bevel (182) determines a transmission ratio between an axial displacement distance of the outer actuating drive (152) and an axial displacement distance of the inner unit (156).

2. Medical instrument of claim 1, **characterized in that** the outer actuating drive (154, 150) comprises a single actuating element (56, 160) which can both be rotated and axially displaced.

3. Medical instrument of claim 1, **characterized in that** the outer actuating drive (106) has two actuating elements (108, 110), a first actuating element (108) for causing the rotation of the inner unit (104) and a second actuating element (110) for causing the axial displacement of the inner unit (104).

4. Medical instrument of claim 3, **characterized in that** the second actuating element (110) for causing the axial displacement of the inner unit (104) is designed rotatable and is in connection with a bushing-like body (118) which causes the rotational movement of the second actuating element (110) into an axial displacement of at least one magnet (128) of the outer actuating drive (106).

5. Medical instrument of claims 3 or 4, **characterized in that** the first actuating element (108) is mounted at the second actuating element (110) in such a manner that turning the first actuating element (108) causes a turning of the inner unit (104) out of any axial displacement position of the outer actuating drive (106).

6. Medical instrument of anyone of claims 1 through 5, **characterized in that** the inner unit (53, 104, 156) can be axially moved between two stops (48, 50; 100, 102), wherein the axial displacement distance (72) of the outer actuating drive (54) is a manifold longer than the displacement distance (74) between the two stops (48, 50; 100, 108).

7. Medical instrument of anyone of claims 1 through 6, **characterized in that** the inner movable unit (53, 104) comprises a bushing (42, 118) supporting the at least one magnet (44, 94) of the inner unit (53, 104).

8. Medical instrument of claim 7, **characterized in that** the outer actuating drive (54, 106, 132, 152) is designed as a control wheel (58) which supports the at least one magnet (64, 128), and **in that** the control wheel (58) surrounds the bushing (42, 92) of the inner unit (53, 104, 156).

9. Medical instrument of anyone of claims 1 through 8, **characterized in that** several magnets (144-147; 158) are disposed in a circumferentially and in an axially distributed manner at the inner unit (134, 15) and **in that** several magnets (138-143; 154) which are distributed circumferentially and axially too are arranged at the outer actuating drive, wherein similar poles of the inner and the outer magnets are opposed one to another, respectively.

10. Medical instrument of anyone of claims 1 through 9, **characterized in that** the axially displacement of the inner unit (156) is against a holding mechanism which holds the inner unit (156) in any axial displacement position.

11. Medical instrument of claim 1, **characterized in that** by acting the bevel (182) of the inner unit with the force of the spring, the at least one ball (188) is pushed against a shoulder (184).

## Revendications

1. Instrument médical, doté d'un organe de réglage extérieur (54, 106, 132, 152) présentant au moins un aimant (64, 128), qui est couplé magnétiquement avec une unité intérieure mobile (53, 104, 134, 156) présentant au moins un aimant (44, 94) et disposée à l'intérieur de l'instrument médical (10, 80, 150), de telle sorte qu'un déplacement de l'organe de réglage extérieur provoque un déplacement de l'unité intérieure mobile, ledit au moins un aimant (64, 128) de l'organe de réglage extérieur (54, 106, 132, 152) étant conçu aussi bien coulissant axialement que rotatif, et en outre l'unité intérieure mobile (53, 104, 132, 156) étant conçue aussi bien coulissante axialement que rotative, le couplage magnétique entre l'organe de réglage extérieur (54, 106, 132, 152) et l'unité intérieure (53, 104, 132, 156) étant conçu de telle sorte qu'une rotation de l'organe de réglage extérieur provoque une rotation de l'unité intérieure et qu'un coulissement axial dudit au moins un aimant (64, 128) dudit organe de réglage provoque un coulissement axial de l'unité intérieure, et la course de coulissement axial de l'organe de réglage extérieur (152) pouvant être transformée par une démultiplication en une course de coulissement de l'unité intérieure (156) plus courte par rapport à elle,
**caractérisé en ce**
**qu'**au moins une bille (188) est disposée entre une surface inclinée (182) de l'unité intérieure (156) et une surface inclinée (192) d'une douille (178) qui l'entoure, laquelle bille (188) est mobile radialement entre les surfaces inclinées (182, 192), et que la pente de la surface inclinée (182) détermine un rapport de démultiplication entre une course de coulissement axial de l'organe de réglage extérieur (152) et une course de coulissement axial de l'unité intérieure (156).

2. Instrument médical selon la revendication 1, **caractérisé en ce que** l'organe de réglage extérieur (54, 150) présente un seul élément de réglage (56, 160) qui est à la fois rotatif et coulissant axialement.

3. Instrument médical selon la revendication 1, **caractérisé en ce que** l'organe de réglage extérieur (106) présente deux éléments de réglage (108, 110), un premier élément de réglage (108) pour produire la rotation de l'unité intérieure (104) et un deuxième élément de réglage (110) pour produire le coulissement axial de l'unité intérieure (104).

4. Instrument médical selon la revendication 3, **caractérisé en ce que** le deuxième élément de réglage (110) pour produire le coulissement axial de l'unité intérieure (104) est conçu rotatif et est en liaison avec un corps en forme de douille (118) qui transforme le mouvement de rotation du deuxième élément de réglage (110) en un coulissement axial dudit au moins un aimant (128) de l'organe de réglage extérieur (106).

5. Instrument médical selon la revendication 3 ou la revendication 4, **caractérisé en ce que** le premier élément de réglage (108) est monté sur le deuxième élément de réglage (110) de telle sorte qu'une rotation du premier élément de réglage (108) provoque une rotation de l'unité intérieure (104) à partir de n'importe quelle position de coulissement axial de l'organe de réglage extérieur (106).

6. Instrument médical selon l'une des revendications 1 à 5, **caractérisé en ce que** l'unité intérieure (53, 104, 156) peut aller et venir axialement entre deux butées (48, 50 ; 100, 102), la course de coulissement axial (72) de l'organe de réglage extérieur (54) étant cependant plusieurs fois plus longue que la course de coulissement (74) entre les deux butées (48, 50 ; 100, 108).

7. Instrument médical selon l'une des revendications 1 à 6, **caractérisé en ce que** l'unité intérieure mobile (53, 104) présente une douille (42, 118) qui porte ledit au moins un aimant (44, 94) de l'unité intérieure (53, 104).

8. Instrument médical selon la revendication 7, **caractérisé en ce que** l'organe de réglage extérieur (54, 106, 132, 152) est réalisé sous la forme d'une molette de réglage (58) qui porte ledit au moins un aimant (64, 128), et que la molette de réglage (58) entoure la douille (42, 92) de l'unité intérieure (53, 104, 156).

9. Instrument médical selon l'une des revendications 1 à 8, **caractérisé en ce que** plusieurs aimants (144-147; 158) sont disposés circonférentiellement et répartis axialement sur l'unité intérieure (134, 156), et que plusieurs aimants (138-143 ; 154) sont également disposés circonférentiellement et répartis axialement sur l'organe de réglage extérieur (132, 152), les pôles identiques des aimants intérieurs et extérieurs se faisant face.

10. Instrument médical selon l'une des revendications 1 à 9, **caractérisé en ce que** le coulissement axial de l'unité intérieure (156) s'effectue contre un mécanisme de maintien qui maintient l'unité intérieure (156) dans n'importe quelle position de coulissement axial.

11. Instrument médical selon la revendication 1, **caractérisé en ce que**, du fait que la surface inclinée (182) de l'unité intérieure peut être soumise à la force d'un ressort, ladite au moins une bille (188) peut être pressée contre un épaulement (184).
